# EUROPEAN PATENT SPECIFICATION

(11) **EP 0 497 878 B1**
(45) Date of publication and mention of the grant of the patent: **10.12.1997**
(21) Application number: 90916656.3
(22) Date of filing: 24.10.1990
(51) Int. Cl.: C12N 9/10, C12N 15/00, C12N 15/54, C12N 15/63, C12P 19/04

(54) **METHOD FOR PRODUCING SECRETABLE GLYCOSYLTRANSFERASES**
METHODE ZUR HERSTELLUNG VON SEKRETIERBAREN GLYCOSYLTRANSFERASEN
PROCEDE DE PRODUCTION DE GLYCOSYLTRANSFERASES SECRETABLES

(30) Priority: 24.10.1989 US 426577
(43) Date of publication of application: 12.08.1992
(73) Proprietor: THE REGENTS OF THE UNIVERSITY OF CALIFORNIA, Oakland, California 94612-3550 (US); AMGEN INC., Thousand Oaks, CA 91320-1789 (US)
(72) Inventor: PAULSON, James, C., Sherman Oaks, CA 941403 (US); UJITA-LEE, Eryn, Redondo Beach, CA 90278 (US); COLLEY, Karen, J., Los Angeles, CA 90025 (US); ADLER, Beverly, Newbury Park, CA 91320 (US); BROWNE, Jeffrey, K., Camarillo, CA 93010 (US); Weinstein, Jasminder, Westlake Village, CA 91361 (US)
(74) Representative: MacGregor, Gordon
(86) International application number: PCT/US90/06088
(87) International publication number: WO 91/06635

(56) References cited:
- WO-A-90/07000
- BIOCHEM. J. vol. 253, 1988, LONDON, ENGL.; pages 577 - 580 U. STICHER ET AL. 'Purification of a alpha2,6-sialyltransferase from rat liver by aye chromatography'
- BIOCHEM. J. vol. 256, no. 2, 1 December 1988, LONDON, ENGL.; pages 623 - 631 G.
- LAMMERS AND J. JAMIESON 'The role of cathepsin D-like activity in the release of Gal-beta1,4GlcNAc alpha2,6-sialyltransferase from rat liver golgi membranes during the acute-phase response'
- J. BIOL. CHEM. vol. 262, no. 36, 25 December 1987, AM. SOC. MOL. BIOL., INC. US; pages 17735 - 17743 J. WEINSTEIN ET AL. 'Primary structure of beta- galactoside alpha2,6-sialyltransferase'
- BIOCHEM. BIOPHYS. RES. COMMUN. vol. 139, no. 1, 29 August 1986, ACADEMIC PRESS, N.Y., US; pages 163 - 168 H.E. APPERT ET AL. 'Isolation of a cDNA coding for human galactosyltransferase'
- J. BIOL. CHEM. vol. 264, no. 23, 15 August 1989, AM. SOC. MOL. BIOL. INC., US; pages 13848 - 13855 E.U. LEE ET AL. 'Alteration of terminal glycosylation sequences on N-linked oligosaccharides of chinese hamster ovary cells by expression of beta-galactoside alpha2,6-sialyltransferase'
- BIOCHEM. BIOPHYS. RES. COMMUN. vol. 157, no. 2, 15 December 1988, ACADEMIC PRESS, N.Y., US; pages 657 - 663 K.A. MASRI ET AL. 'Identification of the full- length coding sequence for human galactosyltransferase (beta-N- acetylglucosaminide: beta1,4-galactosyltransferase)'
- J. BIOL. CHEM. vol. 260, no. 20, 15 September 1985, AM. SOC. MOL. BIOL. INC., US; pages 11223 - 11230 R. FISHER ET AL. 'Isolation and characterization of the human tissue-type plasminogen activator structural gene including its 5' flanking region'
- J.BIOL.CHEM. vol. 264, no. 30, 25 October 1989, AM. SOC. MOL. BIOL. INC., US; pages 17615 - 17618 J.C. PAULSON AND K.J. COLLEY 'Glycosyltranferases'
- J. BIOL. CHEM. vol. 264, no. 30, 25 October 1989, AM. SOC. MOL. BIOL. INC., US; pages 17619 - 17622 K.J. COLLEY ET AL. 'Conversion of a golgi apparatus sialyltransferase to a secretory protein by replacement of the NH2-terminal signal anchor with a signal peptide'
- PROC. NATL. ACAD. SCI. vol. 86, no. 21, November 1989, NATL ACAD. SCI., WASHINGTON, DC, US; pages 8227 - 8231 R.D. LARSEN ET AL. 'Isolation of a cDNA encoding a murine UDPgalactose:beta-D-gala ctosyl-1,4-N-acetyl-D-glucosaminide
- alpha-1,3-galactosyltransferase: Expression cloning by gene transfer'
- Cell, Vol. 41, issued May 1985, G. GIL et al. "Membrane-Bound Domain of HMG CoA Reductase is Required for Sterol-Enhanced Degradation of the Enzyme", see pp. 249-258, particularly p. 249.
- Journal of Biological Chemistry, Vol. 257, No. 22, issued 25 November 1982, J. WEINSTEIN et al. "Purification of a Gal 1 4 Glc NAc 2 6 Sialytransferase and a Gal 1 3(4)Glc NAc 2 3 Sialytransferase to Homogeneity from Rat Liver", see pp. 13835-13844.

## Description

### BACKGROUND OF THE INVENTION

### 1. Field of the Invention

The present invention relates generally to the cellular mechanisms and enzymes involved in the glycosylation of proteins manufactured by the cell. More particularly, the present invention involves altering the enzyme production capabilities of a cell by providing a method for producing a soluble glycosyltransferase which is secreted by the cell and recovered for further use. The present invention also relates to cells which have been genetically altered to produce a soluble, secreted glycosyltransferase ; a vector for use in expressing such a glycosyltransferase in a host cell ; and an expression cassette encoding a secretable, soluble glycosyltransferase.

### 2. Description of Related Art

This invention was made with government support under Grant Contract Nos. GM-27904 and GM-1157 awarded by the National Institute of Health. The government has certain rights in this invention. The publications and other reference materials referred to herein to describe the background of the invention and to provide additional detail regarding its practice are hereby incorporated by reference. For convenience, the reference materials are numerically referenced and grouped in the appended bibliography.

Glycosyltransferases are important enzymes which are essential to'the cellular synthesis of carbohydrates. The glycosyltransferases and their role in enzyme-catalyzed synthesis of carbohydrates are presently being extensively studied (43,44). These enzymes exhibit high specificity for forming carbohydrate structures of defined sequence. Consequently, purified glycosyltransferases are increasingly used as enzymatic catalysts in carbohydrate synthesis. Application of these enzymes has been limited because of difficulties in isolating and purifying them. As a result, glycosyltransferases are only available in very small amounts and are extremely expensive.

The isolation and purification of glycosyltransferases is difficult because of their low abundance in cells and because the enzymes are membrane-bound glycoproteins which reside in the Golgi apparatus of cells. Accordingly, the presently utilized purification procedures involve the use of animal tissues from which the enzymes must be extracted and purified. The standard purification techniques, moreover, typically produce preparations which contain a small but significant percentage of the isolated enzymes which remain membrane bound. Thus, these purification procedures are not amenable to large scale production and therefore are not well suited to meet the present and future demands for purified enzymes to be used in research and in industrial applications involving synthesis of carbohydrates.

As a result, there is presently a need to provide methods for producing increased amounts of purified soluble glycosyltransferases, uncontaminated by membrane bound glycosyltransferases, wherein the method is amenable to relatively large scale production of purified enzymes.

In accordance with, the present invention, a method is provided as is claim 1 for producing catalytically active glycosyltransferases which are soluble and readily secreted from cells. The secretion of relatively large quantities of such catalytically active enzymes provides a simplified procedure for purifying and recovering the glycosyltransferases.

The present invention also provides a composition as set out in Claim 2; a vector as set out in Claim 5; and an expression cassette as set out in Claim 7.

The method of the present invention involves genetically altering a cell so that it produces, instead of the normal membrane-bound glycosyltransferase, a soluble glycosyltransferase which is readily secreted by the cell. The secreted glycosyltransferase is then readily recovered, preferably by conventional procedures for use in industrial applications and research involving carbohydrate synthesis.

The homogeneous, soluble glycosyltransferases produced by the method of the present invention are generally substantially less contaminated by membrane-bound glycosyltransferases than those produced by standard methods. The soluble glycosyltransferases are suitable for a variety of uses, such as enzymatically synthesizing oligosaccharides on a large scale by, for example, serially reacting a starting oligosaccharide with a succession of soluble glycosyltransferases and the appropriate sugar nucleotides.

The above discussed and many other features and attendant advantages of the present invention will become better understood by reference to the following detailed description when taken in conjunction with the accompanying drawings.

### BRIEF DESCRIPTION OF THE DRAWINGS

Fig. 1 shows the domain structure of the sialyltransferase which is anchored to Golgi apparatus membranes by an NH₂-terminal hydrophobic domain. The stem region is believed to carry the retention signal that keeps the enzyme in the Golgi apparatus.

Fig. 2 compares the essential features of cloned cDNA's to other Golgi apparatus glycosyltransferases, all of which contain NH₂-terminal hydrophobic membrane domains, predicting each to have the same topology as the sialyltransferase (44).

Fig. 3 shows an exemplary construction of a soluble sialyltransferase (sp-ST) in accordance with the present invention as compared to the construction membrane bound sialyltransferase (ST).

Fig. 4 depicts test results showing secretion of the soluble sialyltransferase (sp-ST) by Chinese hamster ovary (CHO) cells.

Fig. 5 shows test results demonstrating that the intracellular form of ST is sensitive to Endo H while the secreted ST is resistant to Endo H.

Fig. 6 depicts test results showing that the soluble sp-ST retains sialyltransferase activity.

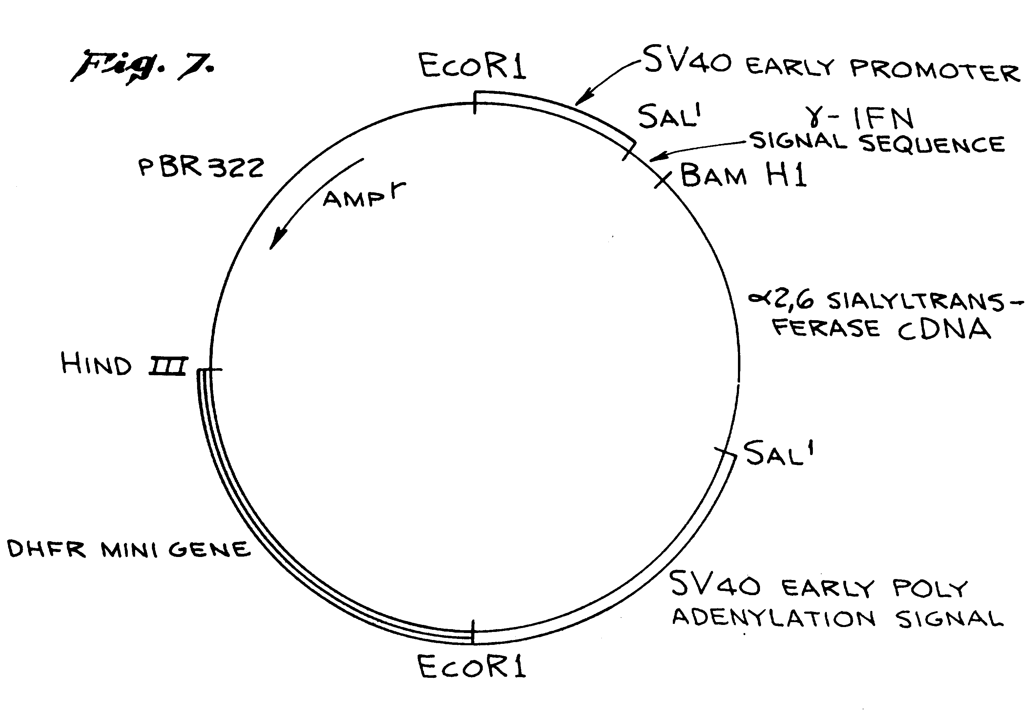

Fig. 7 is a diagram showing the exemplary vector construction used to transfect CHO with the gene expressing the soluble sp-ST protein.

### DETAILED DESCRIPTION OF THE INVENTION

The present invention involves controlling the production of glycosyltransferases in a cell by using genetic engineering to instruct the cell to produce glycosyltransferases which are soluble and can be secreted by the cell. This is accomplished by instructing the cell to produce glycosyltransferases which are lacking both a membrane anchor domain and a retention signal which are believed to normally anchor the glycosyltransferase in the Golgi apparatus of the cell. The cell is instructed to secrete the glycosyltransferase, as opposed to producing it intracellularly, by incorporating in the genetic material and instructions for producing a cleavable secretion-signal peptide. It was discovered that the membrane anchor and retention signal can be removed from glycosyltransferases without destroying the catalytic activity of the enzyme. The resulting soluble peptide is sorted by the cell and transported out of the cell for recovery and further use.

A major consideration in cell biology is sorting and transport of glycosyltransferases and other membrane glycoproteins to their multiple destinations. One hypothesis is that glycoproteins destined for the cell surface are transported by a default pathway and require no routing information, while glycoproteins destined for other subcellular localizations require signals that assist in their sorting and transport (1-3). One well documented transport signal is the mannose 6-phosphate recognition marker found on some lysosomal enzymes (4). These transport signals mediate the enzymes' recognition by mannose 6-phosphate receptors which transport them to the lysosomes as they exit the biosynthetic pathway from the trans-Golgi. In the case of resident proteins of the ER and the Golgi apparatus, the putative signals are viewed as retention signals, since the transport mechanism is likely the same as the default pathway, and the proteins must then resist the flow of the default pathway once they arrive at their site of residence (1-3).

Progress has been made in elucidating the retention signals for the endoplasmic reticulum (ER) retained proteins (6-10). Analysis of deletion mutants and fusion proteins of soluble ER proteins such as protein disulfide isomerase, prolyl hydroxylase, grp78, and grp94 has demonstrated that a COOH-terminal Lys-Asp-Glu-Leu sequence is sufficient for retention in the ER (6,7). In this case a resident Lys-Asp-Glu-Leu receptor protein is postulated for retention of these proteins, although such a receptor has not been identified. Less is known about the retention signals of membrane-bound proteins of the ER (8-10).

Elucidation of the signals for retention of membrane proteins in the Golgi apparatus has been equally refractory. Machamer and Rose (11) have demonstrated that the first of three membrane-spanning regions of the El protein of coronavirus is required for the localization/retention of this viral protein to the cis- to medial-Golgi complex. How this region participates in Golgi apparatus localization is not yet understood.

β-galactoside α2,6-sialyltransferase is a membrane bound glycosyltransferase of the Golgi apparatus which participates in the addition of terminal sialic acid residues to N-linked oligosaccharide chains. Studies have been conducted to identify the structural basis for localization of this enzyme within the cell (14,15). In rat liver hepatocytes, hepatoma cells, and in intestinal globlet cells, β-galactoside α2,6-sialyltransferase has been localized by immunoelectron microscopy to the transcisternae of the Golgi and the trans-Golgi network, whereas in intestinal absorptive cells, the enzyme is more diffusely localized throughout the cisternal stacks (14-16).

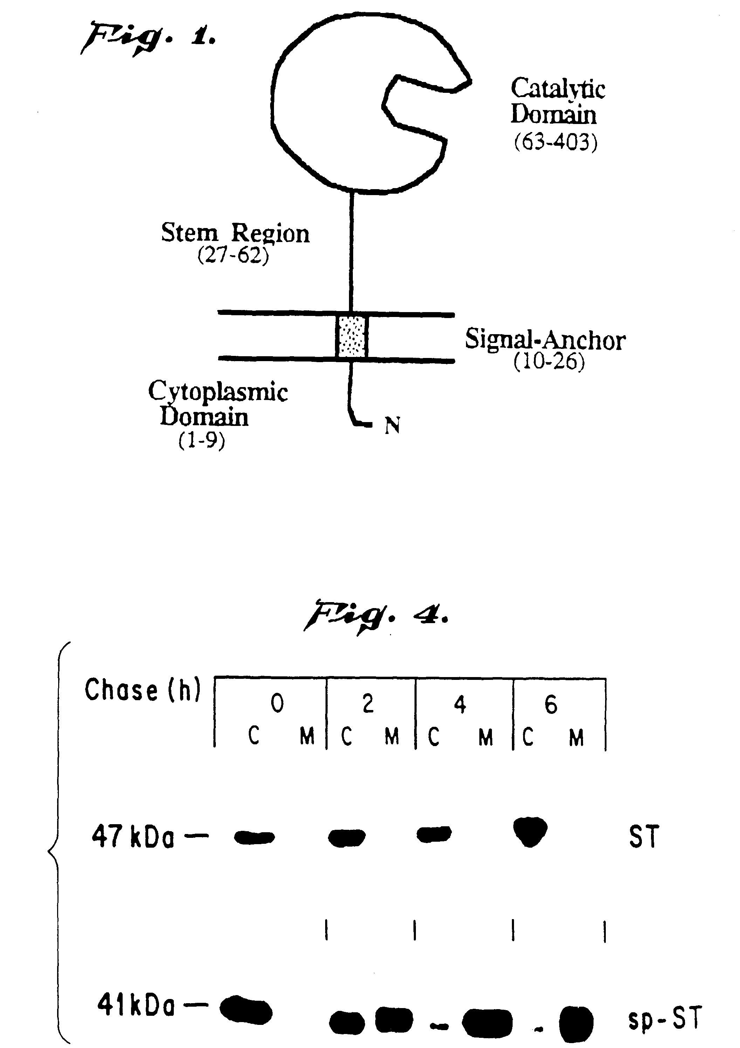

As diagrammed in Fig. 1, the rat liver sialyltransferase is believed to be a class 11 membrane glycoprotein protein with a 9-amino acid NH₂-terminal cytoplasmic tail, a 17-amino acid signal-anchor domain, and a luminal domain which includes an exposed stem region followed by a 41-kDa catalytic domain (19). The existence of an exposed stem region was initially suggested by the purification of a soluble form of the rat liver sialyltransferase which was missing the first 62 amino acids due to proteolytic degradation during isolation. Soluble forms of the sialyltransferase also are found in various secretions and body fluids including milk and colostrum (18) and serum (20,21). The slow release of the sialyltransferase catalytic domain from the transmembrane anchor, through the action of endogenous proteases in the Golgi apparatus or trans-Golgi network, has been proposed to account for the appearance of the soluble enzyme in these fluids (21,22).

The above considerations suggest that sialyltransferase may act like any other secretory protein once released from its NH₂-terminal signal-anchor, providing that the signal for retention in the Golgi apparatus is not part of the catalytic domain of the enzyme. As set forth in more detail below, the present invention is based on the discovery that the NH₂-terminal signal-anchor and stem region of the sialyltransferase can be replaced with a cleavable signal sequence to produce a secretable enzyme which retains its catalytic activity. Proteins transported across the cell membrane typically have an N-terminal sequence rich in hydrophobic amino acids about 15 to 30 amino acids long. Sometime during the process of passing through the membrane, the signal sequence is cleaved by signal peptidase. Watson et al., Molecular Biology of the Gene (The Benjamin/Cummings Publishing Co., Inc., Menlo Park, CA. 1987), which is incorporated herein by reference. Many sources of signal peptides are well known to those skilled in the art and can include, for example, the insulin signal sequence, the tissue plasminogen activator signal sequence, and the like. In general, the N-terminus of essentially any secreted protein is a potential source of a signal sequence suitable for use in the present invention.

In accordance with the present invention, it was found that removal of the sialyltransferase membrane-anchor alone is not sufficient for rapid and efficient secretion of the soluble glycosyltransferase. Construction and expression of a sialyltransferase mutant in which the anchor sequence is replaced by a cleavable signal peptide by recombinant DNA techniques results in a soluble sialyltransferase missing the N-terminal 28 amino acids but containing the intact stem region. This mutant is localized to the Golgi apparatus prior to being slowly secreted from the cell (t_{½}≥24 hours). In contrast, the soluble sialyltransferase construct missing the N-terminal 57 amino acids is rapidly secreted from the cell (t_{½}=2-3 hours). These results show that a retention signal for Golgi apparatus localization resides in the stem region of the sialyltransferase and, in accordance with the present invention, if substantially all of the stem region as well as the signal-anchor domain of the sialyltransferase is removed, rapid and efficient secretion of the soluble glycosyltransferase will be obtained. Preferably, fewer than about ten stem region amino acids remain on the secreted enzyme, more preferably about three, or less.

The number of stem region amino acids that must be removed to eliminate a functional retention signal can be readily determined by, for example, producing different recombinant constructs which have various deletions or modifications of the gene and observing the rate of secretion by a transformed cell. The precise number of stem region amino acids remaining is not critical so long as a functional retention signal is eliminated. A secretable, soluble glycosyltransferase will most preferably have a t_{½} of less than about 12 hours, and ideally between about 2 and about 3 hours. The term "t_{½}" as used herein is defined as the time required for half of the expressed soluble glycosyltransferase to be secreted from the transformed cell.

The present invention basically involves transfecting a host cell with a vector carrying a gene which expresses a glycosyltransferase that has the membrane anchor and most of the stem region replaced with a cleavable secretion signal segment. The resulting soluble glycosyltransferase, when expressed in the cell, is secreted by the cell. The secreted soluble glycosyltransferase is then separated from the cell media for use in industrial applications or carbohydrate synthesis research. Accordingly, the invention provides a useful procedure for producing relatively large amounts of easily recoverable homogeneous, soluble glycosyltransferases which retain their catalytic activity. The term "homogeneous, soluble glycosyltransferase" refers to a soluble glycosyltransferase composition which contains soluble glycosyltransferases substantially free of naturally associated proteins, including membrane bound glycosyltransferases, and/or agents, such as detergents, used to solubilize the enzyme. The term "substantially pure form" indicates at least 90% purity, preferrably 95-99% or more.

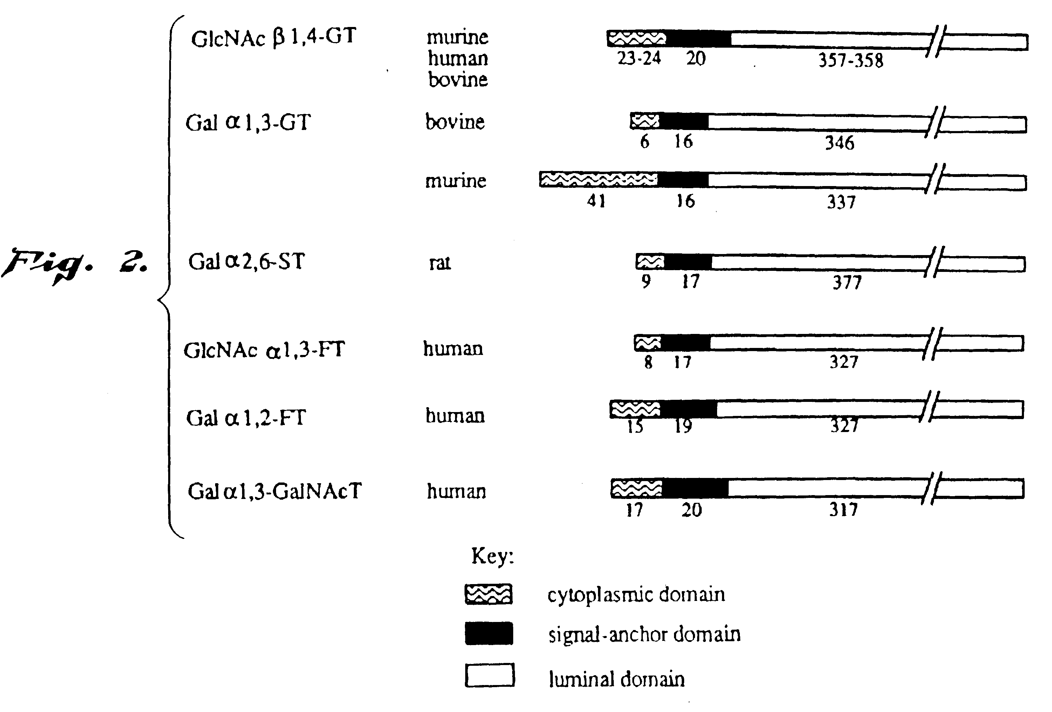

The invention has wide application to the production of glycosyltransferases including N-acetylglucosaminaltransferases, N-acetylgalactosaminyltransferases, sialyltransferases, fucosyltransferases, galactosyltransferases and mannosyltransferases providing that they exhibit similar topology to the sialyltransferase. Indeed, as summarized in Fig. 2 and Table 1, other glycosyltransferase cDNAs cloned to date also exhibit an NH₂-terminal signal-anchor sequence like the sialyltransferase, predicting the same topology found for the sialyltransferase (Fig. 1). The cloning, sequencing and domain structure of glycosyltransferases is known. See, e.g., Creeger, et al., Methods Enzymol., 83:326 (1982); Creeger et al., J. Biol. Chem., 254:804 (1979); Weinstein et al., J. Biol. Chem., 262:17735 (1987); Paulson et al., Biochem. Soc. Trans., 15:618 (1987); Ernst et al., J. Biol. Chem., 264:3436 (1989); and Rajan et al., J. Biol. Chem., 264: (1989); Larsen et al., Proc. National Acad. Sci. USA, 86:8227-8231 (1989); Larsen et al., Proceedings National Academy of Science U.S.A. 86:8227-8231 (1990); and Kukowska - Latallo et al., Genes and Development (1990), all of which are incorporated herein by reference. Other classes of Golgi apparatus enzymes involved in post-translational modifications may also exhibit similar topology, such as sulfotransferases, glycosidases, acetyltransferases, mannosidases. The following description will be limited to the production of a soluble sialyltransferase with it being recognized by those skilled in the art that other glycosyltransferases can also be produced in secretable forms in accordance with the present invention.

**TABLE 1**

| Glycosyltransferase | Donor Substrate | Sequence Formed |
|---|---|---|
| **Galactosyltransferases** | | |
| GlcNAcβ1,4-GT (E.C.2.4.1.38) | UDP-Gal | Galβ1,4GlcNAc-R |
| Gal α1,3-GT (E.C.2.4.1.151) | UDP-Gal | Galα1,3Galβ1,4GlcNAc-R |

| **Sialyltransferase** | | |
|---|---|---|
| Gal α2,6-ST (E.C.2.4.99.1) | CMP-NeuAc | NeuAcα2,6Galβ1, 4GlcNAc-R |

| **Fucosyltransferases** | | |
|---|---|---|
| GlcNAc α1,3-FT (E.C.2.4.1.65) | GDP-Fuc | Fucα1,3 GlcNAc-R Galβ1,4 |
| | | Fucα1,4 GlcNAc-R Galβ1,3 |
| Gal α1,2-FT (E.C.2.4.1.69) | GDP-Fuc | Fucα1,2Galβ1,4GlcNAc-R |
| **N-Acetylgalactosaminyltransferase** | | Fucα1,2Galβ1,3GalcNAc-R |
| Gal α1,3-GalNAcT (Blood group A transferase) | UDP-GalNAc | GalNAcα1,3 Gal-R Fucα1,2 |

The present invention relates to the use of recombinant DNA techniques to produce glycosyltransferases. Applicants believe that all of the techniques (e.g. isolation of nulceic acids, cloning, tranfection, and the like) are standard and well known in the art. The techniques used here generally follow those in Sambrook et al., Molecular Cloning: A Laboratory Manual, (Spring Harbor Laboratory Press, 2nd Ed. 1989), which is incorporated herein by reference.

Generally, to obtain sufficient amounts of a desired glycosyltransferase for large-scale reactions, the enzyme is cloned and expressed as a recombinant soluble enzyme according to the methods described more fully below. Suitable methods include isolation of RNA from a cell known to express the desired enzyme, followed by preparation of a cDNA library as described in Sambrook et al., supra. The desired cDNA is conveniently identified by subtraction using the method of Davis (Handbook of Experimental Immunology, Vol. 2, pgs. 1-13 (1986), which is incorporated herein by reference). The cDNA library is also suitable for probing with, for example, a nondegenerative probe to identify the desired clone. Alternatively, the cDNA library constructed in an expression vector is suitable for probing with an antibody to the desired enzyme. Standard transfection methods are used to produce mammalian or insect cell lines which express large quantities of the desired soluble glycosyltransferase which is then purified using standard techniques. See, e.g., Colley et al., J. Biol. Chem. 264:17619-17622 (1989) and Guide to Protein Purification, Vol. 182, Methods in Enzymology (Deutscher ed., 1990), both of which are incorporated herein by reference.

The host cell which is transfected can be any of the well known cell lines which are capable of producing glycosyltransferases. Exemplary cell lines include Chinese hamster ovary (CHO) cells, mouse L cells, mouse A9 cells, baby hamster kidney cells, C127 cells, PC8 cells, insect cells, yeast (saccharomyces cerevisae) and other eukaryotic cell lines capable of the expression of glycosyltransferases. The particular procedure used to introduce the altered genetic material into the host cell for expression of the soluble glycosyltransferase is not particularly critical. Any of the well known procedures for introducing foreign nucleotide sequences into host cells may be used. These include the use of calcium phosphate transfection, polybrene, protoplast fusion, electroporation, liposomes, microinjection, plasma vectors, viral vectors and any of the other well known methods for introducing cloned genomic DNA, cDNA, synthetic DNA or other foreign genetic material into a host cell (see Sambrook et al., supra). It is only necessary that the particular genetic engineering procedure utilized be capable of successfully introducing at least one gene into the host cell which is capable of expressing the altered glycosyltransferases.

The particular vector used to transport the genetic information into the cell is also not particularly critical. Any of the conventional vectors used for expression of recombinant glycoproteins in eukaryotic cells may be used. Expression vectors containing regulatory elements from eukaryotic viruses are typically used. SV40 vectors include pSVT7 and pMT2. Vectors derived from bovine papaloma virus include pBV-1MTHA, and vectors derived from Epstein Bar virus include pHEBO, and p205. Other exemplary vectors include pMSG, pAV009/A⁺, pMTO10/A ⁺, pMAMneo-5, bacculovirus pDSVE, and any other vector allowing expression of glycoproteins under the direction of the SV-40 early promoter, SV-40 later promoter, metallothionein promoter, murine mammary tumor virus promoter, Rous sarcoma virus promoter, polyhedrin promoter, or other promoters shown effective for expression in eukaryotic cells. For high expression of glycosyltransferases it is desirable to use a vector containing a DHFR gene such as pAV009/A⁺, pMTO10/A⁺ or the exemplary vector pDSVE described in detail below. Such vectors used in combination with CHO cells which are lacking DHFR (e.g., CHO DHFR) allow amplification of the vector DNA carrying the glycosyltransferase gene by selection of the cells with methotrexate. However, other selectable markers resulting in gene amplification such as the sodium, potassium ATPase gene and ouabain selection (45) would be equally suitable. Alternatively, high yield expression systems not involving gene amplification would also be suitable, such as using a bacculovirus vector in insect cells, with the glycosyltransferase under the direction of the polyhedrin promoter (46).

The expression vector of the present invention will typically contain both prokaryotic sequences that facilitate the propagation of the vector in bacteria as well as one or more eukaryotic transcription units that are expressed only in eukaryotic cells, such as mammalian cells. The vector may or may not comprise a eukaryotic replicon. If a eukaryotic replicon is present, then the vector is amplifiable in eukaryotic cells using the appropriate selectable marker. If the vector does not comprise a eukaryotic replicon, no episomal amplification is possible. Instead, the transfected DNA integrates into the genome of the transfected cell, where the promoter directs expression of the desired gene. The expression vector is frequently constructed from elements derived from different, well characterized viral or mammalian genes. For a general discussion of the expression of cloned genes in cultured mammalian cells, see Sambrook et al., supra, Ch. 16.

The prokaryotic elements that are typically included in the mammalian expression vector include a replicon that functions in E. coli, a gene encoding antibiotic resistance to permit selection of bacteria that harbor that recombinant plasmids, and unique restriction sites in nonessential regions of the plasmid to allow insertion of eukaryotic sequences. The particular antibiotic resistance gene chosen is not critical, any of the many resistance genes known in the art is suitable. The prokaryotic sequences are preferably chosen such that they do not interfere with the replication of the DNA in eukaryotic cells.

The expression vector contains a eukaryotic expression cassette that contains all the elements required for the expression of the glycosyltransferase DNA in eukaryotic cells. A typical expression cassette contains a promoter operably linked to the DNA sequence encoding a soluble glycosyltransferase and signals required for efficient polyadenylation of the transcript. The term "operably linked" as used herein refers to linkage of a promoter upstream from a DNA sequence such that the promoter mediates transcription of the DNA sequence. The DNA sequence encoding the glycosyltransferase will be linked to a cleavable signal peptide sequence to promote secretion of the encoded protein by the transformed cell. Additional elements of the cassette may include enhancers and, if genomic DNA is used as the structural gene, introns with functional splice donor and acceptor sites.

Eukaryotic promoters typically contain two types of recognition sequences, the TATA box and upstream promoter elements. The TATA box, located 25-30 base pairs upstream of the transcription initiation site, is thought to be involved in directing RNA polymerase to begin RNA synthesis. The other upstream promoter elements determine the rate at which transcription is initiated.

Enhancer elements can stimulate transcription up to 1,000 fold from linked homologous or heterologous promoters. Enhancers are active when placed downstream from the transcription initiation site. Many enhancer elements derived from viruses have a broad host range and are active in a variety of tissues. For example, the SV40 early gene enhancer is suitable for many cell types. Other enhancer/promoter combinations that are suitable for the present invention include those derived from polyoma virus, human or murine cytomegalovirus, the long term repeat from various retroviruses such as murine leukemia virus, murine or Rous sarcoma virus and HIV. See, Enhancers and Eukaryotic Expression, (Cold Spring Harbor Pres, N.Y. 1983), which is incorporated herein by reference.

In the construction of the expression cassette, the promoter is preferably positioned about the same distance from the heterologous transcription start site as it is from the transcription start site in its natural setting. As is known in the art, however, some variation in this distance can be accommodated without loss of promoter function.

In addition to a promoter sequence, the expression cassette should also contain a transcription termination region downstream of the structural gene to provide for efficient termination. The termination region may be obtained from the same gene as the promoter sequence or may be obtained from different genes. If the mRNA encoded by the structural gene is to be efficiently translated, polyadenylation sequences are also commonly added to the vector construct. Two distinct sequence elements are required for accurate and efficient polyadenylation: GU or U rich sequences located downstream from the polyadenylation site and a highly conserved sequence of six nucelotides, AAUAAA, located 11-30 nucelotides upstream. Termination and polyadenylation signals that are suitable for the present invention include those derived from SV40, or a partial genomic copy of a gene already resident on the expression vector.

In addition to the elements already described, the expression vector of the present invention may typically contain other specialized elements intended to increase the level of expression of cloned genes or to facilitate the identification of cells that carry the transfected DNA. For instance, a number of animal viruses contain DNA sequences that promote the extra chromosomal replication of the viral genome in permissive cell types. Plasmids bearing these viral replicons are replicated episomally as long as the appropriate factors are provided by genes either carried on the plasmid or with the genome of the host cell. Genes encoding selectable markers are also typically used. Suitable selectable markers include, thymidine kinase, aminoglycoside phosphotransferase, hygromycin B phosphotransferase, xanthine-guanine phosphoribosyl transferase, CAD (carbamyl phosphate synthetase, aspartate transcarbamylase, and dihydroorotase), adenosine deaminase, DHFR, and asparagine synthetase.

The recombinantly produced glycosyltransferases can be used to produce specific oligosaccharides. The term "oligosaccharide" refers to a compound sugar or sugar moiety that yields two to ten monosaccharides upon hydrolysis. A "monosaccharide" is a single sugar unit that cannot be hydrolyzed into smaller units, it is typically a pentose, a hexose, or larger molecule. The starting oligosaccharide may be an oligosaccharide moiety on a glycoconjugate, such as a glycoprotein or a glycolipid or an unconjugated oligosaccharide. It may also be attached to a carrier moiety or to an activatable intermediate that will allow attachment to suitable carrier moiety. The starting oligosaccharide typically comprises up to ten monosaccharides but may also be a single monosaccharide to which monosaccharides are added in succession by the approprjate glycosyltransferase to produce the desired oligosaccharide. The oligosaccharides are preferably prepared on a large scale so that economically efficient synthesis of a variety of oligosaccharides is feasible. For a general discussion of enzyme-catalyzed synthesis of oligosaccharides, see Toone et al., Tetrahedron 45: 5365-5422 (1989), and Sabesan et al., J. Am. Chem. Soc., 108: 2068-2280 (1986), both of which are incorporated herein by reference.

Typically, the desired oligosaccharide is synthesized by sequentially reacting a starting oligosaccharide with a series of soluble glycosyltransferases. Each enzymatic reaction for each glycosyltransferase uses the appropriate nucleotide sugar as a donor substrate. As used herein, the term "nucleotide sugar" refers to the activated form of sugars which are transferred to the oligosaccharide. These molecules are nucleoside phosphate sugars, such as UDP-Glc, UDP-GLcUA, UDP-GLcNAc, UDP-Gal, UDP-GalNAc, GDP-Man, GDP-Fuc, and CMP-NeuAc, and the like. The glycosyl transfer reactions are preferably carried out with added alkaline phosphotase (such as that from calf intestine, CIAP) in order to consume the nucleoside phosphate byproduct which may inhibit the reaction.

The nomenclature used to describe the oligosaccharides follows the conventional nomenclature. Standard abbreviations for individual monosaccharides are used. For instance, 2-N-acetylglucosamine is represented by GlcNAc, 5-N-acetylneuraminic acid (a sialic acid) is NeuAc, fucose is Fuc, galactose is Gal, and glucose is Glc. Unless otherwise indicated, all sugars are D-isomers in the cyclic configuration. The two anomers of the cyclic forms are represented by α and β.

The monosaccharides are generally linked by glycosidic bonds to form oligo- and polysaccharides. The orientation of the bond with respect to the plane of the rings is indicated by α and β. The particular carbon atoms that form the bond between the two monosaccharides are also noted. Thus, a Bglycosidic bond between C-1 of galactose and C-4 of glucose is represented by Galβl,4Glc. For the D-sugars (e.g. D-GlcNAc, D-Gal, and D-NeuAc) the designation α means the hydroxyl attached to C-1 (C-2 in NeuAc) is below the plane of the ring and β is above the ring. In the case of L-fucose, the α designation means the hydroxyl is above the ring and β means it is below.

The following examples of the present invention describe the transfection of CHO cells with genes capable of expressing β-galactoside α2,6-sialyltransferase (ST) in a modified form wherein the membrane anchor and retention signal located at the NH₂ terminal region of the sialyltransferase are replaced with the cleavable signal peptide of human gamma-interferon. The cleavable signal sequence is required for targeting the modified glycosyltransferase to the secretory machinery of the cell (endoplasmic reticulum, Golgi apparatus, etc.), but the choice of the cleavable sequence is also not particularly critical. Other cleavable sequences could be used such as the insulin signal sequence (47), the tissue plasminogen activator signal sequence (used in the commercial vector pMAMneo-S) or any other cleavable signal sequence. Thus it will be understood that the principles disclosed with respect to the expression and secretion of soluble sialyltransferase (sp-ST) by CHO cells also applies to the other various glycosyltransferases, host cells, vectors and cleavable signal sequences previously mentioned.

The following example demonstrates the production and recovery of a secretable sialyltransferase which is compared to membrane-bound sialyltransferase. The example is as follows:

### Materials

The pECE expression vector used to express the membrane-bound sialyltransferase was obtained from Dr. William J. Rutter (University of California at San Francisco School of Medicine-Department of Biochemistry and Biophysics) (23), and CHO cells were obtained from Dr. L. Shapiro (University of California at Los Angeles School of Medicine). Tran ³⁵S-label (85% methionine and 15% cysteine) (>1000Ci/mmol) was purchased from ICN Biomedicals, Inc. (Irvine, California), and τ-³²P-ATP(1000µCi/ml) was obtained from New England Nuclear (Boston, Massachusetts). Restriction enzymes used in fusion protein construction were obtained from Bethesda Research Laboratories (Indianapolis, Indiana) and International Biotechnology Incorporated (New Haven, Connecticut). All cell culture reagents were obtained from IBCO (Grand Island, New York). Endo-β-N-acetylglucosaminidase H (Endo H) in 10 mmol/l phosphate buffer was purchased from Boehringer Mannheim Biochemicals (Indianapolis, Indiana). Immunoprecipititin was obtained from Bethesda Research Laboratories (Gaithersburg, Maryland) and prepared according to the manufacturer's directions. Dimethyl sulfoxide was purchased from Sigma (St. Louis, Missouri) and 2,5 diphenyl oxazole was obtained from Aldrich (Milwaukee, Wisconsin). Methotrexate was purchased from Sigma (St. Louis, Missouri). The expression vector used for expressing the soluble sialyltransferase was pDSVE (described in U.S. Patent Applications Ser. Nos. 025,344 and 152,045, the contents of which are hereby incorporated by reference).

### Construction of the signal peptide-sialyltransferase (sp-ST) fusion protein

To generate a secreted sialyltransferase, the 5' 169 nucleotides (through amino acid 57) of the sialytransferase coding sequence in the ST3 cDNA were replaced with a synthetic gene segment coding for the 23 amino acid signal peptide and first three amino acids of human gamma interferon yielding the fusion gene product shown in Fig. 1. The sialyltransferase coding sequence in the ST3 cDNA was first modified by the introduction of a unique BamHl site at nucleotides 163-171 (amino acids 55-57) and a unique *Sall* site in the 3' untranslated region at nucleotides 1395-1400 by site-directed mutagenesis (24). The modified *BamH-1 Sall* sialyltransferase gene fragment was isolated and ligated through the *BamH1* site, with the synthetic gamma interferon signal peptide gene fragment and inserted into the pDSVE eukaryotic expression vector. Expression of the sialyltransferase gene is driven by the SV40 early gene promoter and uses the SV40 early gene polyadenylation signal. In addition, the vector contains a mouse dihydrofolate reductase (DHFR) gene as a selectable marker (25).

### Expression of the membrane-bound and soluble sialyltransferases in CHO cells

The membrane-bound α2,6 sialyltransferase was expressed via the transfection of CHO DHFR cells with the pECE vector. Isolation of a stably transfected CHO cell clone expressing the membrane-bound α2,6 sialyltransferase from the SV40 early promoter of the pECE vector has been described by Lee et al. (26). The secreted sialyltransferase expression vector was introduced into a CHO DHFR cell line by the calcium phosphate microprecipitation method (27), modified as described (28,29). Following selection by growth in media with dialyzed serum pools of stably transfected CHO cells were obtained, and were further selected with methotraxate stepwise to 0.3 µM.

### Pulse-chase analysis and immunoprecipitation of sialyltransferase proteins

CHO cells stably expressing either the wild type sialyltransferase or the sp-ST fusion protein were grown on 100 mm plastic dishes until confluent and then incubated with methionine-free D-MEM for one hour. Media was removed, cells were washed and 2.5 ml methionine-free D-MEM with 100mCi/ml Trans ³⁵S-label was added to each dish. After one hour the media was removed, cells were washed with phosphate buffered saline (PBS) and then incubated for various times up to 24 hours with α-MEM, 5% fetal bovine serum (chase period). Following the chase period, media was collected, cells washed in PBS and lysed with 2.5 ml of immunoprecipitation buffer (50 mM Tris-HCl, pH 7.5, 150 mM NaCl, 5 mM EDTA, 0.5% Nonidet P-40, 0.1% SDS).

Sialyltransferase protein was immunoprecipitated from media and cell lysales at each time point. Eight µl of preimmune rabbit serum was incubated with 500 µl of sample for 15 minutex at 25°C. Eighty microliters of a 10% suspension of *S. aureus* in immunoprecipitation buffer was added to each tube, and incubation was continued for an additional 15 minutes. *S. aureus*-immune complexes were pelleted by centrifugation at 16,000 x g for 1 minute. Supernatants were transferred to a new tube and incubated with 8 µl affinity purified rabbit anti-rat a 2,6 sialyltransferase antibody for approximately 16 hours at 4°C. Again, 80 µl of a 10% suspension of *S. aureus* was added to each tube and incubation was continued for 30 minutes at 25°C. Samples were always subjected to end-over-end rotation during all of the previously described incubations. *S. aureus*-immune complexes were pelleted as described above and pellets washed three times with immunoprecipitation buffer and once with 10 mM Tris-HCl, pH 7.5, 0.1% SDS. Sialyltransferase protein was eluted from *S. aureus* pellets by boiling 5 minutes in 40 µl 1x Laemmli gel sample buffer (10% glycerol, 2% SDS, 65 mM Tris-HCl, pH 7.5, 0.5 mg/ml bromphenol blue, 10% β-mercaptoethanol). Immunoprecipitated proteins were electrophoresed on 10% polyacrylamide gels according to the method of Laemmli (30). Radiolabeled proteins were visualized by fluorography using 2,5 diphenyloxazole/dimethyl sulfoxide (31) and gels were exposed to Kodak XAR-5 film at -80°C.

### Endo-β-N-acetylglucosaminidase H digestions

After the final wash of the *S. aureus* pellets, immunoprecipitated proteins were treated essentially as described in Dahms et al. (32). Following acetone precipitation, protein pellets were resuspended in 20 µl Endo H buffer (0.1 M sodium citrate, pH 6.0, 0.075% SDS, 0.2% β -mercaptoethanol), 2 mU Endo H was added and the digests were incubated at 37°C for 16 hours. To stop the digestions, 20 µl of a 2x gel sample buffer was added to each tube and then samples were boiled for 5 minutes in preparation for SDS-polyacrylamide gel electrophoresis.

### CDP-hexanolamine-Agarose Affinity Chromatography

Affinity chromatography of unlabeled and radiolabeled CHO cell media was performed on small columns (1.5 ml) of CDP-hexanolamine-agarose essentially as described by Weinstein et al. (33). Columns were equilibrated with Buffer E (10 mM Na cacodylate, pH 6.5, 0.1% Triton CF-54, 0.15 M NaCl) and after application of media (1.25 ml diluted with 1.25 ml of Buffer E) columns were developed by application of 1.2 ml Buffer E, 4.2 ml Buffer H (25 mM Na cacodylate, pH 5.3, 0.1% Triton CF-54, 0.15M NaCl) and 3 ml Buffer H plus 1 mM CDP *(Elute, 1 mM CDP).* Fractions of 300 µl were collected and assayed for total protein using the Pierce BCA protein detection assay and for sialyltransferase either by direct enzyme activity or by immunoprecipitation of ³⁵S-labeled sialyltransferase protein followed by SDS polyacrylamide gel electrophoresis.

### Sialyltransferase Assay

Sialyltransferase assays were performed essentially as described previously (33) using CMP-[¹⁴C] NeuAc (285000 cpm/nmole) as a donor substrate and asialo α₁ acid glycoprotein (50 µg) as the acceptor substrate. Observed activity in the elution fractions was corrected for the inhibition produced by the presence of CDP in the assay. Activity is reported as cpm transferred/10 µl/hr.

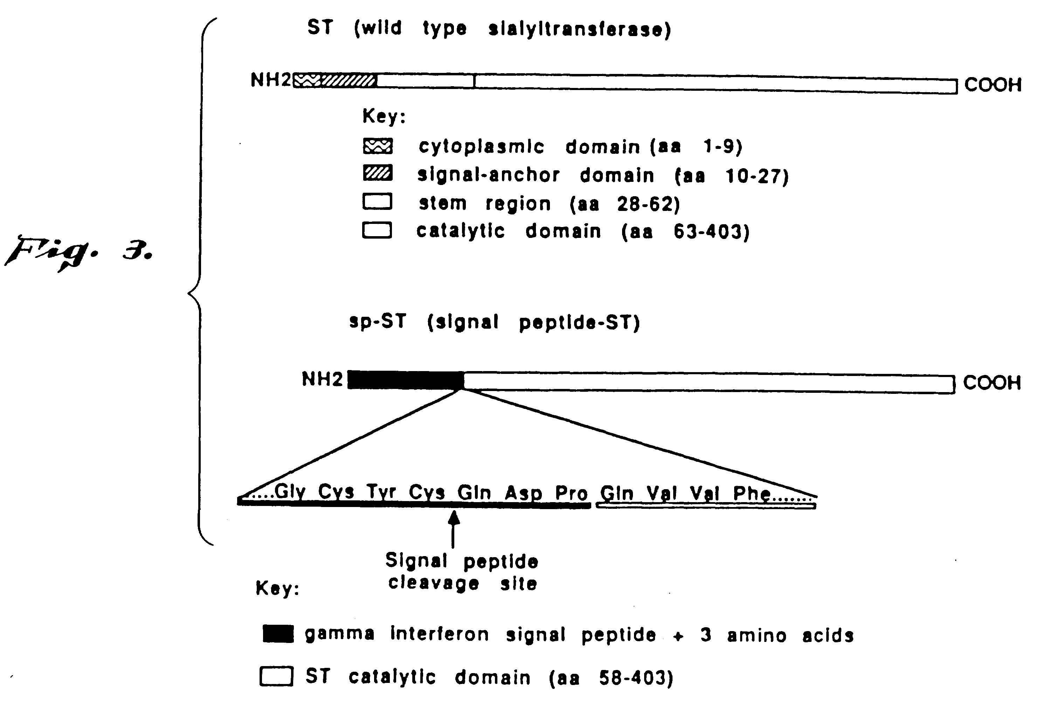

In Fig. 3, the structure of the ST (wild type) sialyltransferase is compared to the fusion sialylt:ransferase (sp-ST). The fusion protein or signal peptide-sialyltransferase (sp-ST), consists of residues 1-26 of human gamma interferon including the cleavable signal peptide (34), and amino acids 58-403 of the sialyltransferase. The NH₂-terminal cytoplasmic tail, signal-anchor domain and most of the putative stem region of the wild type sialyltransferase are missing, however, the sp-ST enters the secretory pathway using the gamma interferon signal peptide. CHO cells were transfected with either the wild type sialyltransferase cDNA (26) or the sp-ST cDNA and stably expressing CHO cell clones were isolated as described above.

The wild type (ST) sialyltransferase (47 kDa) has previously been demonstrated to be catalytically active when expressed in stably transfected CHO cells which are lacking an endogenous β-galactoside α2,6 sialyltransferase (26). The above described pulse-chase analysis and immunoprecipitation of sialyltransferase protein from cell lysates and media of CHO cells revealed that the wild type sialyltransferase remains cell associated for up to 6 hours of chase and is not detected in the media for up to 24 hours (see Fig. 4). In contrast to the wild type sialyltransferase, the sp-ST fusion protein construct is rapidly secreted from CHO cells. At least 50% of the total sp-ST protein is secreted within 2 hours of chase, following a 1 hour labeling period (see Fig. 4). Moreover, the apparent molecular weight of the sp-ST (41 kDa) is consistent with the cleavage of the 2.5 kDa gamma interferon signal peptide.

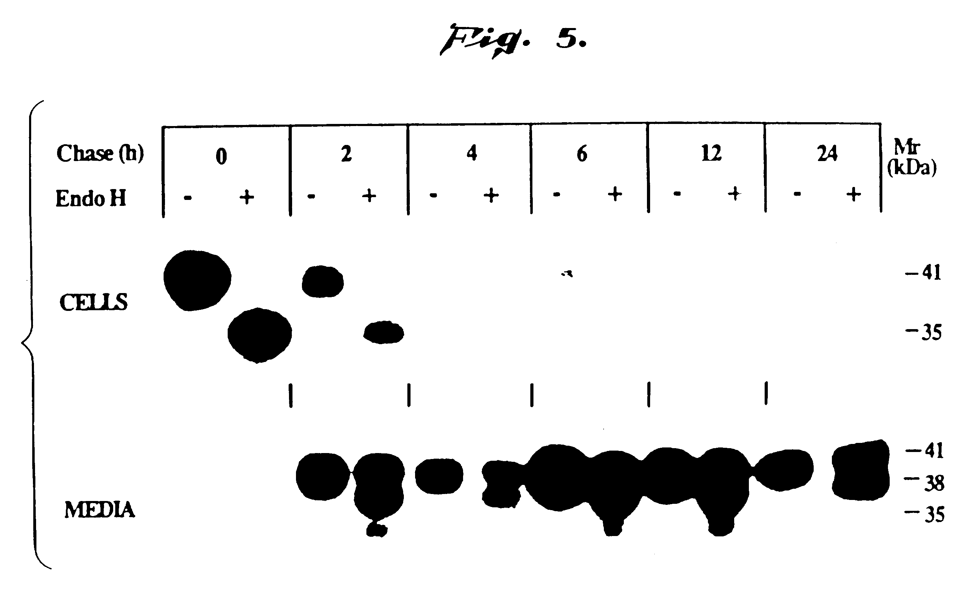

Endo-β-N acetylglucosaminidase H (Endo H) preferentially cleaves high mannose N-linked oligosaccharides and can be used to trace the movement of secretory proteins from the ER to *medial*-Golgi apparatus where processing reactions convert the sugar chains to Endo H resistant forms (2,35). Endo H sensitivity of the N-linked oligosaccharides of both the intracellular and secreted forms of the sp-ST, which contains two N-linked sugar chains, is shown in Fig. 5. The intracellular form of the sp-ST protein (41 kDa) is predominantly Endo H sensitive and yields a single band with an apparent molecular weight of 35 KDa consistent with the cleavage or both N-linked carbohydrate groups. In contrast, the secreted sp-ST fusion protein is largely resistant to Endo H yielding 38 kDa and 41 kDa bands, indicating the resistance of one or both N-linked carbohydrate groups to enzyme digestion, respectively. Because these resistant forms of the sp-ST do not accumulate intracellularly, the results suggest that the rate limiting step in the secretion of the sp-ST is in the protein's migration from ER to the Golgi apparatus. Once the sp-ST oligosaccharides are processed in the Golgi apparatus, the sp-ST protein is rapidly secreted.

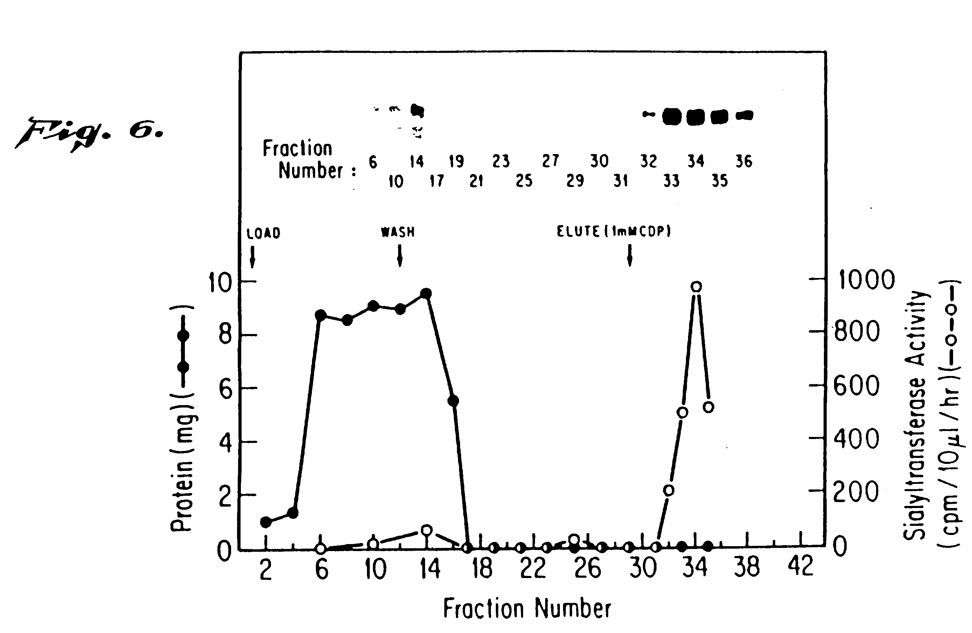

As with the wild type sialyltransferase, the CHO cells expressing the sp-ST synthesize the product of the enzyme detected by the FITC-SNA lectin which shows that the soluble sialyltransferase exhibits catalytic activity during transit through the cell. Moreover, direct enzyme assays detected sialyltransferase activity in the media of CHO cells secreting the sp-ST protein, but not in the media of parental CHO cells or CHO cells expressing the wild type sialyltransferase. This shows that the soluble enzyme is active once secreted from the cells. The ability of sp-ST to bind to the affinity absorbent CDP-hexanolamine-agarose used in the purification of the rat liver sialyltransferase was tested (33). As demonstrated in Fig. 6, sialyltransferase activity in the CHO cell media is bound to the column and elutes with the free ligand CDP. Moreover, the bulk of the immunopreciptable ³⁵S-labeled sp-ST accumulated throughout a 24 hour chase period is also bound to the column and is specifically eluted with CDP. Taken together, these results demonstrate that the secreted enzyme is catalytically active and is stable in the media.

The same principles illustrated in the above example can be extended to the production of any Golgi apparatus glycosyltransferase or other processing enzyme (glycosidase, sulfotransferase, acetyltransferase etc.) as a secretory protein providing that the enzyme has an NH₂-terminal signal-anchor sequence and has the Golgi retention signal in the NH₂-terminal peptide sequence including the cytoplasmic tail, membrane anchor and stem regions which are not required for catalytic activity. All Golgi apparatus glycosyltransferases whose cDNAs have been cloned meet this criterion (Fig. 2 and Table 1). In addition, a Golgi glycosidase, mannosidase II, has also been shown to have a similar domain structure to the exemplary sialyltransferase illustrated in Fig. 1 (48).

All publications and patent applications referred to above and in the bibliography below are incorporated herein by reference to the same extent as if each individual reference was specifically and individually incorporated herein by reference.

### BIBLIOGRAPHY

1. Pfeffer, S. and Rothman, J.E. (1987) Annu. Rev. Biochem. 56, 829-852.
2. Wieland, F.T., Gleason, M.L., Serafini, T.A., and Rothman, J.E. (1987) Cell 50, 289-300.
3. Klausner, R.D. (1989) Cell 57, 703-706.
4. von Figura, K. and Hasilik, A. (1986) Annu. Rev. Biochem. 55, 167-193.
5. Kornfeld, S. (1986) J. Clin, Invst. 77, 1-6.
6. Munro, S. and Pelham, H.R. B. (1987) Cell 48, 899-907.
7. Pelham, H.R.B. (1988) EMBO J. 7, 913-918.
8. Paabo, S., Bhat, B.M., Wold, W.S.M., and Peterson, P.A. (1987) Cell 50, 311-317.
9. Poruchynsky, M.S. and Atkinson, P.H. (1988) J. Cell Biol. 107, 1697-1706.
10. Stirzaker, S.C. and Both, G.W. (1989) Cell 56, 741-747.
11. Machamer, C.E. and Rose, J.K. (1987) J. Cell Biol. 105, 1205-1214.
12. Fleischer, B. (1981) J. Biol. Chem. 89, 246-255.
13. Kornfeld, R. and Kornfeld, S. (1985) Annu. Rev. Biochem. 54, 631-664.
14. Roth, J., Taatjes, D.J., Lucoq, J.M., Weinstein, J., and Paulson, J.C. (1985) Cell 43, 287-295.
15. Roth, J., Taatjes, D.J., Weinstein, J., Paulson, J.C., Greenwell, P., and Watkins, W.M. (1986) J. Biol. Chem. 261, 14307-14312.
16. Taatjes, D.J., Roth, Weinstein, J., and Paulson J.C. (1988) J. Biol. Chem. 263, 6302-6309.
17. Weinstein, J., Lee, E.U., McEntee, K., Lai, P.H., and Paulson, J.C. (1987) J. Biol. Chem. 263, 17735-17743.
18. Paulson, J.C., Beranek, W.E., and Hill, R.L. (1977) J. Biol. Chem. 252, 2356-2362.
19. Hudgin, R.L. and Schachter, H. (1971) Can. J. Biochem. 49, 829-837.
20. Kaplan, H.A., Woloski, B.M.R.N.J., Hellman, M., and Jamieson, J.C. (1985) J. Biol. Chem. 258, 11505-11509.
21. Lammers, G. and Jamieson, J.C. (1988) Biochem. J. 256, 623-631.
22. Paulson, J.C. Weinstein, J., Ujita, E.L., Riggs, K.J., and Lai, P.H. (1987) Biochem. Soc. Trans. 15, 618-620.
23. Ellis, L., Clauser, E., Morgan, D.O., Edery, M., Roth, R.A., and Rutter, W.A. (1986) Cell 45, 721-732.
24. Zoller, J.J. and Smith, M. (1983) Meth. Enzymol. 100, 468-500.
25. Gasser, C.S., Simonsen, C.C., Schilling, J.W., and Schimke, R.T. (1982) Proc. Natl. Acad. Sci. USA 79, 6522-6526.
26. Lee, E.U., Roth, J., and Paulson, J.C. (1989) J. Biol. Chem., in press.
27. Graham, F.L. and van der Eb, A.J. (1973) Virology, 52, 456-467.
28. Wigler, M., Pellicer, A., Silverstein, S., and Axel, R. (1978) Cell 41, 725-731.
29. Lewis, W.H., Srinivasan, P.R., Siokoc, N., and Siminovitch, L. (1980) Somatic Cell Genetics 6, 333-347.
30. Laemmli, U.K. (1970) Nature 227, 680-685.
31. Bonner, W.M. and Lasky, R.A. (1974) Eur. J. Biochem. 46, 83-88.
32. Dahms, N.M., Lobel, P., Breitmeyer, J., Chirgwin, J.M., and Kornfeld, S. (1987) Cell 50, 181-192.
33. Weinstein, J. de Souza-e-Silva, U., and Paulson, J.C.
(1982) J. Biol. Chem. 257, 13835-13844.
34. Gray, P. and Goeddel, D.V. (1982) Nature 298, 859-863.
35. Maley, F. and Trimle, R.B. (1981) J. Biol. Chem. 256, 1088-1090.
36. Roth, J. and Berger, E.G. (1982) J. Cell Biol. 93, 223-229.
37. Shaper, N.L., Hollis, G.F., Douglas, J.G., Kirsch, I.R., and Shaper, J.H. (1988) J. Biol. Chem. 263, 10420-10428.
38. Nakazawa, K., Ando, T., Kimura, T., and Narimatsu, H. (1988) J. Biochem. (Tokyo) 104, 165-168.
39. Masri, K.A., Appert, H.E., and Fukuda, M.N. (1988) Biochem. Biophys. Res. Comm. 157, 657-663.
40. Berger, E.G. and Hesford, F.J. (1985) Proc. Natl. Acad. Sci. USA 82, 4736-4739.
41. Duncan, J.R. and Kornfeld, S. (1988) J. Cell Biol. 106, 617-628.
42. Beyer, T.A., Sadler, J.E., Rearick, J.I., Paulson, J.C., and Hill, R.L. (1981) Adv.Enzymol. 52, 23-175.
43. Toone, E.J., Simon, Es., Bednarski, M.D., and Whitesides, G.M. (1989) Tetrahed. Rep., in press.
44. Colley, K.M., Lee, E.U., Adler, B., Browne, J.K. and Paulson, J.C. (1989) J. Biol. Chem. 264, in press.
45. Ash, J.F., Fineman, R.M., Kalka, T., Morgan, M. and Wire, B. (1984) J. Cell Biol. 99, 971-983.
46. Smith, G.E., Summers, M.D. and Fraser, M.J. (1983) Mol. Cell Biol. 3, 2156-2165.
47. Hsueh, E.C., Holland, E.C., Carrera, G.M., and Drickamer, K. (1986) J. Biol. Chem. 261, 4940-4947.
48. Moreman, K.W. (1989) Proc. Nat. Acad. Sci. USA 85, 5276-5280.

## Claims

1. A method for producing a soluble glycosyltransferase enzyme which is secreted by a cell, wherein the naturally occurring enzyme includes a retention signal and a membrane anchor, said method comprising the steps of:
introducing into said cell a gene which is capable of expressing a soluble and secretable glycosyltransferase enzyme which lacks said retention signal and anchor domain, but includes a cleavable secretion signal;
expressing said soluble and secretable enzyme in said cell wherein said cell secretes said soluble enzyme; and
recovering the soluble enzyme secreted by said cell.

2. A composition of matter comprising cells which have been genetically altered to produce a soluble glycosyltransferase, wherein the naturally occurring glycosyltransferase includes a retention signal and a membrane anchor, the said cells having been genetically altered by introducing a gene into said cells which is capable of expressing a soluble and secretable glycosyltransferase that lacks said retention signal and membrane anchor domain, but includes a cleavable secretion signal.

3. A method as in Claim 1 or a composition as in Claim 2, wherein said gene is introduced into said cell by transfection with a vector comprising DNA which codes for said secretable glycosyltransferase.

4. A method or composition as in any preceding claim wherein the cell into which said gene is introduced is selected from the group consisting of Chinese hamster ovary cells, mouse L cells, Mouse A9 cells, baby hamster kidney cells, C127 cells, PC8 cells and insect cells.

5. A vector for use in expressing a soluble glycosyltransferase in a host cell, wherein the naturally occurring glycosyltransferase includes a retention signal and a membrane anchor, said vector comprising DNA having a nucleotide sequence which codes for a glycosyltransferase which lacks the membrane anchor and retention signal, but includes a cleavable secretion signal.

6. A method or composition as in Claim 3 or a vector as in Claim 4, wherein said vector is selected from the group consisting of pECE, pMSG, pAV009/A+, pMT010/A+, pMAMucc-S, bacculovirus and pDSVE.

7. An expression cassette comprising a promoter operably linked to a DNA sequence encoding a secretable, soluble glycosyltransferase, wherein the naturally occurring glycosyltransferase includes a retention signal and a membrane anchor, said sequence encoding a glycosyltransferase that lacks said retention signal and membrane anchor, but includes a cleavable secretion signal.

8. A method or composition as in Claim 3 or 6, a vector as in Claim 5 or 6, or an expression cassette as in Claim 7, wherein said DNA is a cDNA sequence.

9. The method, composition, vector or expression cassette according to any preceding claim wherein said gene or DNA encodes a glycosyltransferase having a γ-cleavable secretion signal peptide substituted for the membrane anchor and retention signal, said cleavable signal peptide being selected from the group consisting of γ-interferon signal peptide, insulin signal peptide and TPA signal peptide.

10. A method, composition, vector or expression cassette as in Claim 9, wherein said DNA sequence encoding the cleavable secretion signal is a human γ-interferon signal sequence.

11. A method, composition, vector or expression cassette according to any preceding claim, wherein said glycosyltransferase is selected from the group consisting of N-acetylglucosaminyltransferases, N-acetylgalactosaminyltransferases,sialyltransferases,fucosyltransferases, galactosyltransferases, mannosyltransferases, sulfoltransferases, acetyltransferases and mannosidases.

12. A method, composition, vector or expression cassette according to any preceding claim, wherein said glycosyltransferase is β-galactoside α2,6-sialyltransferase.

13. A method, composition, vector or expression cassette as in any preceding claim wherein said gene or DNA sequence encodes a glycosyltransferase which consists essentially of a catalytic domain, about three stem region amino acid residues, and said cleavable secretion signal.

14. A cell comprising the expression cassette of any one of Claims 7 to 13.

15. The cell of Claim 14 which is a mammalian cell or an inset cell.

## Patentansprüche

1. Verfahren zur Herstellung eines löslichen und von einer Zelle sezernierten Glycosyltransferaseenzyms, wobei das natürlich vorkommende Enzym ein Retentionssignal und einen Membrananker enthält, durch
Einführen eines Gens mit der Fähigkeit zur Expression eines löslichen und eines sezernierbaren Glycosyltransferaseenzyms ohne das Retentionssignal und die Ankerdomäne, jedoch mit einem abspaltbaren Sezernierungssignal in die betreffende Zelle,
Exprimieren des löslichen und sezernierbaren Enzyms in der betreffenden Zelle, wobei die betreffende Zelle das lösliche Enzym sezerniert und
Gewinnen des durch die Zelle sezernierten löslichen Enzyms.

2. Zusammensetzung, umfassend Zellen, die zur Produktion einer löslichen Glycosyltransferase genetisch verändert wurden, wobei die natürlich vorkommende Glycosyltransferase ein Retentionssignal und einen Membrananker enthält und wobei die betreffenden Zellen durch Einführen eines Gens mit der Fähigkeit zur Expression einer löslichen und einer sezernierbaren Glycosyltransferase ohne das Retentionssignal und die Membranankerdomäne, jedoch mit einem abspaltbaren Sezernierungssignal in die betreffenden Zellen genetisch verändert wurden.

3. Verfahren nach Anspruch 1 oder Zusammensetzung nach Anspruch 2, wobei das Gen in die Zelle durch Transfektion mit einem DNA mit Codierung für die betreffende sezernierbare Glycosyltransferase umfassenden Vektor eingeführt wurde.

4. Verfahren oder Zusammensetzung nach einem der vorhergehenden Ansprüche, wobei die Zelle, in die das Gen eingeführt wurde, aus der Gruppe Eierstockzellen des chinesischen Hamsters, Maus-L-Zellen, Maus-A9-Zellen, Nierenzellen vom Babyhamster, C127-Zellen, PC8-Zellen und Insektenzellen ausgewählt ist.

5. Vektor zur Verwendung bei der Expression einer löslichen Glycosyltransferase in einer Wirtzelle, wobei die natürlich vorkommende Glycosyltransferase ein Retentionssignal und einen Membrananker enthält und wobei der Vektor DNA mit einer Nukleotidsequenz mit Codierung für eine Glycosyltransferase ohne einen Membrananker und das Retentionssignal, jedoch mit einem abspaltbaren Sezernierungssignal umfaßt.

6. Verfahren oder Zusammensetzung nach Anspruch 3 oder Vektor nach Anspruch 4*, wobei der Vektor aus pECE, pMSG, pAV009/A+, pMT010/A+, pMAMucc-S, Bacculovirus und pDSVE ausgewählt ist.

7. Expressionskassette mit einem operativ an eine DNA-Sequenz mit Codierung für eine sezernierbare, lösliche Glycosyltransferase gebundenen Promotor, wobei die natürlich vorkommende Glycosyltransferase ein Retentionssignal und einen Membrananker enthält und wobei die Sequenz für eine Glycosyltransferase ohne das Retentionssignal und den Membrananker, jedoch mit einem abspaltbaren sezernierbaren Signal codiert.

8. Verfahren oder Zusammensetzung nach Anspruch 3 oder 6, Vektor nach Anspruch 5 oder 6 oder Expressionskassette nach Anspruch 7, wobei die DNA aus einer cDNA-Sequenz besteht.

9. Verfahren, Zusammensetzung, Vektor oder Expressionskassette nach einem der vorhergehenden Ansprüche, wobei das Gen oder die DNA für eine Glycosyltransferase mit einem γ-abspaltbaren Sezernierungssignalpeptid als Ersatz für den Membrananker und das Retentionssignal codiert, wobei das abspaltbare Signalpeptid aus der Gruppe γ-Interferonsignalpeptid, Insulinsignalpeptid und TPA-Signalpeptid ausgewählt ist.

10. Verfahren, Zusammensetzung, Vektor oder Expressionskassette nach Anspruch 9, wobei die DNA-Sequenz mit Codierung für das abspaltbare Sezernierungssignal aus einer menschlichen γ-Interferonsignalsequenz besteht.

11. Verfahren, Zusammensetzung, Vektor oder Expressionskassette nach einem der vorhergehenden Ansprüche, wobei die Glycosyltransferase aus der Gruppe N-Acetylglucosaminyltransferasen, N-Acetylgalactosaminyltransferasen, Sialyltransferasen, Fucosyltransferasen, Galactosyltransferasen, Mannosyltransferasen, Sulfoltransferasen, Acetyltransferasen und Mannosidasen ausgewählt ist.

12. Verfahren, Zusammensetzung, Vektor oder Expressionskassette nach einem der vorhergehenden Ansprüche, wobei die Glycosyltransferase aus β-Galactosid-α2,6-sialyltransferase besteht.

13. Verfahren, Zusammensetzung, Vektor oder Expressionskassette nach einem der vorhergehenden Ansprüche, wobei das Gen oder die DNA-Sequenz für eine Glycosyltransferase codiert, die im wesentlichen aus einer katalytischen Domäne, etwa drei Stammregionaminosäureresten und dem abspaltbaren Sezernierungssignal besteht.

14. Zelle mit der Expressionskassette nach einem der Ansprüche 7 bis 13.

15. Zelle nach Anspruch 14, bei es sich um eine Säugetier- oder Insektenzelle handelt.

## Revendications

1. Procédé de production d'une enzyme soluble de type glycosyltransférase sécrétée par une cellule, l'enzyme naturelle comprenant un signal de rétention et une ancre à membrane, ce procédé comprenant les étapes consistant :
à introduire dans ladite cellule un gène capable d'exprimer une enzyme de type glycosyltransférase soluble et sécrétable ne comportant pas ledit signal de rétention et ledit domaine d'ancrage, mais comprenant un signal de sécrétion clivable ;
à exprimer ladite enzyme soluble et sécrétable dans ladite cellule, ladite cellule sécrétant ladite enzyme soluble ; et
à récupérer l'enzyme soluble sécrétée par ladite cellule.

2. Composition de matière comprenant des cellules ayant été génétiquement modifiées pour produire une glycosyltransférase soluble, la glycosyltransférase naturelle comportant un signal de rétention et une ancre à membrane, lesdites cellules ayant été génétiquement modifiées par introduction d'un gène dans ces cellules, capable d'exprimer une glycosyltransférase soluble et sécrétable ne comportant pas ledit signal de rétention et ledit domaine d'ancrage sur membrane, mais comprenant un signal de sécrétion clivable.

3. Procédé selon la revendication 1 ou composition selon la revendication 2, ledit gène étant introduit dans ladite cellule, par transfection à l'aide d'un vecteur comprenant un ADN codant pour ladite glycosyltransférase sécrétable.

4. Procédé ou composition selon l'une quelconque des revendications précédentes, dans lequel la cellule, dans laquelle ledit gène est introduit, est choisie à partir du groupe comprenant les cellules d'ovaire de hamster chinois, les cellules L de souris, les cellules A9 de souris, les cellules de rein de jeune hamster, les cellules C127, les cellules PC8 et les cellules d'insecte.

5. Vecteur destiné à être employé pour exprimer une glycosyltransférase soluble dans une cellule-hôte, la glycosyltransférase naturelle comprenant un signal de rétention et une ancre à membrane, ledit vecteur comprenant un ADN comportant une séquence nucléotidique codant pour une glycosyltransférase ne comportant pas l'ancre à membrane et le signal de rétention, mais comprenant un signal de sécrétion clivable.

6. Procédé ou composition selon la revendication 3, ou vecteur selon la revendication 4, dans lequel ledit vecteur est choisi à partir du groupe comprenant pECE, pMSG, pAV009/A+, pMT010/A+, pMAMucc-S, le bacculovirus et pDSVE.

7. Cassette d'expression comprenant un promoteur fonctionnellement lié à une séquence d'ADN codant pour une glycosyltransférase soluble et sécrétable, la glycosyltransférase naturelle comprenant un signal de rétention et une ancre à membrane, ladite séquence codant pour une glycosyltransférase ne comportant pas ledit signal de rétention et ladite ancre à membrane, mais comprenant un signal de sécrétion clivable.

8. Procédé ou composition selon la revendication 3 ou 6, vecteur selon la revendication 5 ou 6, ou cassette d'expression selon la revendication 7, dans lequel ledit ADN est une séquence d'ADNc.

9. Procédé, composition, vecteur ou cassette d'expression selon l'une quelconque des revendications précédentes, dans lequel ledit gène ou ledit ADN code pour une glycosyltransférase comportant un peptide signal de sécrétion γ-clivable à la place de l'ancre à membrane et du signal de rétention, ledit peptide signal clivable étant choisi à partir du groupe comprenant le peptide signal de l'interféron γ, le peptide signal de l'insuline et le peptide signal du TPA.

10. Procédé, composition, vecteur ou cassette d'expression selon la revendication 9, dans lequel ladite séquence d'ADN codant pour le signal de sécrétion clivable est une séquence signal de l'interféron γ humain.

11. Procédé, composition, vecteur ou cassette d'expression selon l'une quelconque des revendications précédentes, dans lequel ladite glycosyltransférase est choisie à partir du groupe comprenant les N-acétylglucosaminyltransférases, les N-acétylgalactosaminyltransférases, les sialyltransférases, les fucosyltransférases, les galactosyltranférases, les mannosyltransférases, les sulfoltransférases, les acétyltransférases et les mannosidases.

12. Procédé, composition, vecteur ou cassette d'expression selon l'une quelconque des revendications précédentes, dans lequel ladite glycosyltransférase est la β-galactoside α-2,6-sialyltransférase.

13. Procédé, composition, vecteur ou cassette d'expression selon l'une quelconque des revendications précédentes, dans lequel ledit gène ou ladite séquence d'ADN code pour une glycosyltransférase consistant essentiellement en un domaine catalytique, en environ trois résidus d'aminoacide de région formant tronc, et ledit signal de sécrétion clivable.

14. Cellule comprenant la cassette d'expression de l'une quelconque des revendications 7 à 13.

15. Cellule selon la revendication 14, qui est une cellule de mammifère ou une cellule d'insecte.
